# FASCICULE DE BREVET EUROPEEN

(11) **EP 3 482 379 B1**
(45) Date de publication et mention de la délivrance du brevet: **22.04.2020**
(21) Numéro de dépôt: 17740702.0
(22) Date de dépôt: 07.07.2017
(51) Int. Cl.: G08B 21/24, A61C 13/00

(54) **SYSTÈME ET PROCÉDÉ DE TRAÇABILITÉ D'UNE PROTHÈSE DENTAIRE, ET PROTHÈSE DENTAIRE CORRESPONDANTE.**
SYSTEM UND VERFAHREN ZUR RÜCKVERFOLGBARKEIT EINES ZAHNERSATZES SOWIE ENTSPRECHENDER ZAHNERSATZ
SYSTEM AND METHOD FOR TRACEABILITY OF A DENTAL PROSTHESIS, AND CORRESPONDING DENTAL PROSTHESIS

(30) Priorité: 08.07.2016 FR 1656595
(43) Date de publication de la demande: 15.05.2019
(73) Titulaire: Université d'Angers, 49000 Angers (FR); Centre Hospitalier Universitaire d'Angers, 49100 Angers (FR)
(72) Inventeur: BARTHELAIX, Annick, 72430 Asniere sur Vegre (FR); RIO, Jocelyn, 49000 Angers (FR); SPIESSER, Antoine, 49000 Angers (FR)
(74) Mandataire: Vidon Brevets & Stratégie
(86) Numéro de dépôt international: PCT/EP2017/067143
(87) Numéro de publication internationale: WO 2018/007614

(56) Documents cités:
- WO-A1-98/49660
- US-A1- 2010 289 646
- US-B1- 6 239 705

## Description

### 1. Domaine de l'invention

Le domaine de l'invention est celui des prothèses dentaires, et plus particulièrement des prothèses dentaires amovibles, qu'il s'agisse de prothèses dentaires intégrales ou partielles (dentiers à crochet). Plus précisément, l'invention concerne la traçabilité de telles prothèses dentaires, tant en termes de localisation, d'identification, que de suivi de leur entretien, etc.

### 2. Art antérieur et ses inconvénients

De nombreuses difficultés associées au port de prothèses dentaires amovibles sont recensées, notamment, mais non exclusivement, chez les personnes dépendantes, et plus particulièrement, mais non seulement, en milieu institutionnel.

Parmi ces difficultés, un problème fréquent est celui de la perte de ces prothèses dentaires, qui peuvent être égarées par leur propriétaire. Si, à domicile, il peut s'avérer fastidieux de retrouver le lieu insolite dans laquelle la prothèse dentaire a pu être oubliée, cette problématique est bien plus complexe lorsque le propriétaire vit en milieu institutionnel. En effet, la zone de recherche de la prothèse égarée est bien plus vaste. En outre, lorsqu'une prothèse dentaire est retrouvée, il peut s'avérer difficile d'identifier son propriétaire parmi l'ensemble des pensionnaires de l'institution. Enfin, cette perte pose également des problèmes d'hygiène.

On note par ailleurs qu'un mauvais entretien de la prothèse dentaire peut être à l'origine de la perte ultérieure de cette dernière. En effet, une prothèse dentaire mal entretenue peut causer des lésions dans la bouche du patient, une douleur, ou une simple gêne, qui peuvent pousser ce dernier à l'enlever, et à la laisser dans un endroit plus ou moins insolite.

De plus, si l'on ne retrouve pas rapidement la prothèse et son propriétaire, cette perte peut avoir des conséquences importantes en termes de coût de remplacement de la prothèse (conséquences financières), mais aussi en termes de confort pour l'utilisateur, chez qui l'absence de prothèse peut occasionner des troubles plus ou moins sévères de l'alimentation, un déficit nutritionnel, et également une dégradation de l'image de soi, et de celle qu'il offre aux autres.

Pour pallier ces différents inconvénients, le document de brevet WO 02/13719 A1 propose d'insérer dans les prothèses dentaires amovibles des étiquettes d'identification radiofréquence passives, de type RFID. De telles étiquettes stockent en mémoire un code, qui peut prendre la forme d'un identifiant. Elles ne nécessitent pas d'alimentation électrique, mais utilisent l'énergie des signaux électromagnétiques reçus d'un lecteur d'étiquettes pour émettre le code ou l'identifiant qu'elles contiennent. Cet identifiant s'affiche alors sur un écran du lecteur, qui peut être connecté à une base de données de l'institution, mémorisant en correspondance l'ensemble des identifiants, et les données d'identification de leurs propriétaires.

En outre, un tel lecteur portatif peut également être utilisé pour localiser les prothèses dentaires égarées. Pour ce faire, le lecteur portatif émet un signal électromagnétique destiné à exciter l'étiquette RFID intégrée dans la prothèse dentaire, et comprend un récepteur, qui détecte le signal émis en réponse par la prothèse dentaire, lorsqu'elle se trouve à portée du lecteur portatif.

Cette solution, bien qu'intéressante pour identifier le propriétaire d'une prothèse égarée, est peu satisfaisante pour résoudre le problème de la localisation d'une prothèse perdue. En effet, l'utilisation d'une puce dite passive, sous forme d'étiquette RFID, restreint fortement le champ de recherche de cette prothèse, la portée du lecteur d'étiquette RFID étant limitée à quelques dizaines de centimètres. Ainsi, cette solution technique n'est notamment pas adaptée, et insuffisante, dans l'exemple particulier d'un utilisateur vivant en milieu institutionnel, qui aurait égaré sa prothèse dentaire en salle de restauration, et pour lequel le personnel soignant remarquerait cette perte lorsque l'utilisateur se trouve de retour dans sa chambre.

Dans un autre domaine applicatif, le document de brevet US 6,239,705 propose une technique alternative qui consiste à intégrer dans une prothèse dentaire une puce active, qui peut émettre des signaux radiofréquence de plus grande portée, pour permettre de localiser son propriétaire. Cette solution ne vise pas à résoudre le problème technique de la perte des prothèses dentaires, mais celui de la localisation de leurs propriétaires (dont on considère qu'ils gardent toujours la prothèse en bouche), à des fins d'applications militaires notamment. La prothèse dentaire intègre également une alimentation, sous forme de pile ou de batterie, pour fournir l'énergie nécessaire à la puce active.

Si elle offre l'avantage d'une plus grande portée de localisation de la prothèse dentaire, l'utilisation d'un émetteur radiofréquence actif pose le problème de l'autonomie de la pile ou de la batterie alimentant la puce active.

Le document de brevet WO 98/49660 propose quant à lui une prothèse dentaire équipée d'une puce active, qui peut émettre un signal sonore ou lumineux pour aider à la localisation de la prothèse lorsqu'elle est égarée. Pour éviter la gêne qui pourrait être occasionnée pour le porteur en cas d'activation de la fonction de localisation lorsque la prothèse est portée en bouche, il est prévu d'équiper la prothèse d'un capteur de présence en bouche. Ainsi, lorsque le capteur détecte que la prothèse se trouve dans la bouche de son utilisateur, l'émetteur de signaux sonores ou lumineux reste dans un état inactif. Le fonctionnement d'un tel capteur est cependant également consommateur en énergie.

Il existe donc un besoin d'une technique de traçabilité d'une prothèse dentaire qui ne présente pas ces différents inconvénients de l'art antérieur.

Notamment, il existe un besoin d'une technique de traçabilité d'une prothèse dentaire qui permette de localiser une prothèse dentaire égarée dans un rayon d'environ une dizaine de mètres ou plus. Il existe également un besoin d'une telle technique qui permette d'identifier aisément le propriétaire d'une prothèse dentaire retrouvée. Il existe enfin un besoin d'une telle technique qui permette de proposer une prothèse dentaire qui présente une autonomie énergétique de plusieurs mois.

### 3. Exposé de l'invention

L'invention répond à ce besoin en proposant un système de traçabilité d'une prothèse dentaire comprenant la prothèse dentaire, dans lequel la prothèse dentaire comprend une source d'alimentation électrique couplée à un émetteur de signaux de radiocommunications apte à prendre un état faiblement actif, dans lequel il émet périodiquement des signaux à une première fréquence d'émission, et un état fortement actif, dans lequel il émet périodiquement des signaux à une deuxième fréquence d'émission supérieure à la première. La prothèse comprend au moins un capteur de présence de la prothèse dans une bouche d'un utilisateur, apte à délivrer une information de présence ou d'absence de la prothèse dans la bouche, et l'émetteur passe de l'état faiblement actif à l'état fortement actif lorsque le capteur délivre une information d'absence de la prothèse dans la bouche. En outre, le système de traçabilité selon un mode de réalisation de l'invention comprend une base formant support de la prothèse dentaire lorsqu'elle n'est pas portée par un utilisateur. **L'**émetteur est configuré pour prendre l'état faiblement actif sur détection d'une présence de la prothèse dentaire à proximité de la base.

Ainsi, l'invention repose sur une approche tout à fait nouvelle et inventive de la traçabilité des prothèses dentaires amovibles. En effet, la prothèse dentaire selon l'invention intègre une puce active, alimentée électriquement, comprenant un émetteur de signaux de radiocommunication, qui peut émettre des signaux d'une portée d'une dizaine de mètres environ, lorsque la prothèse dentaire est égarée. Une telle prothèse dentaire résout donc efficacement le problème de la localisation des prothèses égarées, notamment en milieu institutionnel, grâce à une portée d'émission de signaux de radiocommunication beaucoup plus importante que selon les techniques de l'art antérieur reposant sur l'utilisation d'étiquettes d'identification radiofréquences passives.

En outre, cet émetteur de signaux de radiocommunications est avantageusement couplé à un capteur de présence de la prothèse dentaire dans la bouche de son utilisateur. De cette façon, l'émetteur n'envoie des signaux de radiocommunication selon une fréquence d'émission élevée, et donc consommatrice en énergie, permettant la localisation de la prothèse dentaire, que lorsque celle-ci est effectivement égarée, i.e. lorsque le capteur de présence détecte que la prothèse ne se trouve plus dans la bouche de son utilisateur.

Ainsi, la consommation électrique liée à l'émission de signaux de radiocommunications à forte fréquence par la prothèse dentaire est strictement limitée aux périodes pendant lesquelles la prothèse est égarée, ce qui permet une autonomie énergétique de la prothèse dentaire satisfaisante, par exemple de plusieurs mois.

En outre, un tel système de traçabilité comprend une base formant support de la prothèse dentaire lorsqu'elle n'est pas portée par un utilisateur. La prothèse dentaire est ainsi avantageusement appariée à une base, sur laquelle l'utilisateur doit poser sa prothèse dentaire amovible dès qu'il ne la porte pas : ce support constitue un lieu de rangement privilégié de la prothèse dentaire, et permet d'éviter que cette dernière ne soit égarée, car posée dans un endroit incongru quand l'utilisateur la retire de sa bouche.

Pour réduire encore la consommation énergétique de la prothèse, et ainsi accroître son autonomie, l'émetteur de signaux de radiocommunication de la prothèse reste dans un état faiblement actif tant qu'il est situé à proximité de la base. On notera que, dans une variante de réalisation, cet état faiblement actif est un état inactif, dans lequel la première fréquence d'émission est nulle, et l'émetteur de la prothèse n'émet pas de signaux.

Selon un mode de réalisation, sur détection d'une présence de la prothèse dentaire à proximité de la base, la prothèse est configurée pour fonctionner en mode « base », dans lequel le capteur de présence est dans un état inactif et l'émetteur de signaux de radiocommunication est dans un état faiblement actif.

On réduit ainsi fortement la consommation en énergie de la prothèse dentaire. En effet, lorsque la prothèse dentaire est positionnée sur sa base, il n'est pas nécessaire que le capteur de présence procède à des mesures de présence de la prothèse dans la bouche de son utilisateur. L'émetteur peut également être faiblement actif, voire en variante inactif. Ainsi, lorsqu'un échange de requêtes et réponses entre les émetteurs/récepteurs de la base et de la prothèse confirme la présence de la prothèse dentaire sur son support, il est possible de désactiver ces deux éléments. Selon un premier mode de réalisation, c'est la base qui envoie à la prothèse dentaire une commande de désactivation de ces deux éléments. Selon un second mode de réalisation, c'est la prothèse qui pilote la désactivation de ces deux éléments, sur réception d'une réponse de la base à une requête qu'elle lui a adressée. La consommation d'énergie de la prothèse dentaire est donc fortement réduite, ce qui permet de prolonger l'autonomie de la source d'alimentation électrique de la prothèse.

Selon un mode de réalisation, sur détection d'une absence de la prothèse dentaire à proximité de la base, la prothèse dentaire est configurée pour fonctionner en mode « reconnaissance », dans lequel le capteur de présence opère une mesure de présence à une fréquence déterminée, et dans lequel l'émetteur de signaux de radiocommunications est dans l'état faiblement actif.

Ainsi, dès qu'on détecte que la prothèse dentaire s'éloigne de la base, on active le capteur de présence, afin que celui-ci procède à des mesures régulières de la présence de la prothèse dans la bouche de son utilisateur. En effet, lorsque la prothèse dentaire quitte la base, le scénario le plus probable est qu'elle soit positionnée dans la bouche de son utilisateur : il convient alors de vérifier régulièrement cette présence de la prothèse en bouche. L'émetteur de signaux de radiocommunication peut en revanche rester dans un état faiblement actif, pour réduire la consommation d'énergie de la prothèse, tant que le capteur ne délivre pas une information d'absence de la prothèse dentaire de la bouche de son utilisateur.

La fréquence des mesures opérées par le capteur de présence peut être figée lors de la conception de la prothèse, ou être réglable, par exemple pour s'adapter aux habitudes du propriétaire de la prothèse, ou pour optimiser la consommation d'énergie du capteur.

Selon un mode de réalisation, l'émetteur est configuré pour passer, lorsque le capteur délivre une information d'absence de la prothèse dans la bouche, de l'état faiblement actif à l'état fortement actif dans lequel il est configuré pour émettre périodiquement des signaux à une deuxième fréquence d'émission. La base comprend un module d'émission d'une commande d'un mode de fonctionnement de la prothèse dentaire, qui est configuré pour envoyer à la prothèse dentaire, sur action d'un utilisateur, une commande de fonctionnement en mode « localisation», dans lequel l'émetteur de signaux de radiocommunication est configuré pour émettre périodiquement des signaux à une troisième fréquence d'émission supérieure à la deuxième.

Ainsi, dès que le capteur de présence signale que la prothèse dentaire ne se trouve plus dans la bouche de l'utilisateur, l'émetteur de signaux de radiocommunication passe dans un état fortement actif, et émet périodiquement, mais à une fréquence relativement faible, des signaux de radiocommunication (par exemple une trame BLE toutes les deux minutes). On réduit ainsi la consommation d'énergie de l'émetteur, tant que l'utilisateur n'est pas dans une phase de recherche active de la prothèse dentaire égarée. En effet, la prothèse dentaire peut avoir quitté la bouche de l'utilisateur mais ne pas pour autant être égarée (par exemple, être en cours de nettoyage).

En revanche, dès que l'utilisateur (i.e. le propriétaire de la prothèse, ou un proche, ou un personnel soignant) détecte que la prothèse a été égarée, et souhaite la localiser, il peut activer un mode « localisation », par exemple par actionnement d'un bouton prévu sur la base. La base émet alors une commande de passage en mode « localisation », dans lequel l'émetteur de signaux de radiocommunications de la prothèse va émettre plus fréquemment des signaux (par exemple une trame BLE toutes les 30 secondes), pour permettre à la base de localiser rapidement la prothèse égarée.

Les deuxième et troisième fréquences d'émission de signaux de radiocommunication de la prothèse dentaires sont de préférence choisies pour atteindre un compromis entre une rapidité de localisation de la prothèse égarée et une consommation énergétique maîtrisée de la prothèse dentaire.

La base à laquelle la prothèse dentaire est appariée peut donc piloter le mode de fonctionnement des composants de la prothèse dentaire, et notamment du capteur de présence et de l'émetteur de signaux radiofréquence, en leur envoyant des commandes, par exemple selon le standard Bluetooth® Low Energy (BLE) ou Bluetooth Smart®.

Selon un mode de réalisation, une telle base comprend un module d'alerte configuré pour émettre une alerte :
- lorsqu'une durée de présence de ladite prothèse dentaire à proximité de ladite base est supérieure à un seuil de présence déterminé, ou
- lorsqu'une durée d'absence de ladite prothèse dentaire à proximité de ladite base est supérieure à un seuil d'absence déterminé.

Ainsi, la base permet avantageusement un suivi de l'hygiène de la prothèse dentaire et de son utilisation par son propriétaire.

En effet, lorsque la prothèse reste trop longtemps sur la base, cela peut signifier que la prothèse dentaire n'est pas assez portée : la durée de présence peut être mesurée entre deux éloignements successifs de la prothèse dentaire de son support, ou être évaluée sous forme cumulée, sur une période d'observation donnée (par exemple, la durée de présence cumulée de la prothèse dentaire sur sa base au cours d'une semaine).

De même, si la période entre deux repositionnements successifs de la prothèse dentaire sur sa base est trop longue, cela peut signifier que l'utilisateur n'entretient pas assez sa prothèse dentaire.

Dans chacun de ces deux cas, les conséquences en bouche pour l'utilisateur de la prothèse dentaire peuvent être néfastes, et il peut donc être nécessaire d'émettre une alerte. Cette alerte peut être émise directement par la base. En variante, la base peut remonter ces informations de suivi de durée de présence ou d'absence vers un serveur, qui peut diffuser ces alertes vers un téléphone intelligent ou une tablette des personnes concernées (par exemple le personnel soignant, ou les proches de l'utilisateur). Lorsque la prothèse dentaire n'est pas suffisamment portée, cela peut résulter d'un inconfort pour l'utilisateur, voire d'une blessure des muqueuses buccales provoquée par la prothèse dentaire. L'alerte émise peut alors avantageusement être associée à des conseils donnés à l'utilisateur ou à ses proches sur les réglages de la prothèse dentaire à opérer en cabinet dentaire (réglage de l'articulé dentaire, rebasage/réajustage de la résine au contact des tissus...).

Selon un mode de réalisation, la base comprend une unité de localisation de la prothèse dentaire, comprenant au moins une antenne directive connectée à un module radio aptes à recevoir les signaux de radiocommunication émis par l'émetteur de ladite prothèse dentaire, et un ensemble d'indicateurs lumineux et/ou sonores de contrôle d'une direction de réception desdits signaux.

Ainsi, la base peut avantageusement être utilisée comme appareil de localisation de la prothèse égarée. Pour ce faire, elle est de préférence équipée d'une batterie rechargeable permettant son autonomie énergétique au cours des ces phases de recherche de prothèse. En outre, elle est équipée d'une ou plusieurs antennes directives, par exemple sous forme d'un réseau d'antennes, lui permettant de capter les signaux de radiocommunication émis par la prothèse dentaire égarée, et d'en identifier la direction d'émission. Cette ou ces antennes peuvent par exemple prendre la forme de patchs céramiques. En outre, la base présente de préférence un ensemble d'indicateurs lumineux, par exemple sous la forme d'un panneau de LEDS, permettant d'aider l'utilisateur dans sa recherche de la prothèse, en matérialisant la direction de réception des signaux en provenance de la prothèse. En variante, des bips sonores peuvent être émis par la base, par exemple de fréquence variable selon l'orientation de la base par rapport à la prothèse : la fréquence des bips sonores émis par la base augmente lorsque la base pointe dans la direction de la prothèse dentaire égarée.

Selon un mode de réalisation, la base comprend un module de chargement par induction de ladite source d'alimentation électrique.

Ainsi, repositionner la prothèse dentaire sur sa base permet de recharger la batterie embarquée dans la prothèse.

Selon un mode de réalisation de l'invention, le capteur est un capteur de température, qui délivre une information d'absence lorsque la température mesurée est inférieure à environ 35°C.

En effet, tant que la prothèse est en bouche, la température mesurée par le capteur de présence est sensiblement égale à la température du corps humain, soit de l'ordre de 37°. Une chute de cette température mesurée peut indiquer que la prothèse dentaire ne se trouve plus dans la bouche de son utilisateur, mais a été égarée, par exemple oubliée sur un plateau repas en salle de restauration.

Dans d'autres modes de réalisation de l'invention, un tel capteur de présence peut être par exemple un capteur d'humidité : en effet, tant que la prothèse dentaire est en bouche, elle est en contact avec la salive de l'utilisateur, donc en milieu humide. Une chute de l'hygrométrie mesurée par le capteur de présence peut également indiquer que la prothèse dentaire n'est plus portée par son utilisateur, et peut donc avoir été égarée.

Selon un mode de réalisation de l'invention, la prothèse dentaire comprend une étiquette d'identification radiofréquence passive, et la base comprend un lecteur d'étiquette d'identification radiofréquence apte à détecter une présence ou une absence de la prothèse dentaire à proximité de la base.

Une telle étiquette RFID comprend de préférence des données d'identification de la prothèse dentaire, et de son utilisateur, telles que par exemple :
- un numéro d'identification de la prothèse dentaire ;
- le nom/prénom de son propriétaire, ainsi que son numéro de sécurité sociale ;
- dans le cas d'une vie en milieu institutionnel, le numéro de chambre du propriétaire de la prothèse.

Lorsque la base lit les informations contenues dans l'étiquette RFID de la prothèse, ces informations peuvent s'afficher sur un écran prévu sur la base, ou sur l'écran d'un ordinateur, d'une tablette ou d'un téléphone intelligent (en anglais « smartphone ») auxquels la base est connectée.

Ceci permet de vérifier l'identité du propriétaire de la prothèse dentaire, ce qui s'avère particulièrement utile lorsque la prothèse dentaire a été perdue, puis retrouvée, dans une structure collective (centre hospitalier, maison de retraite, établissement d'hébergement pour personnes âgées dépendantes (EHPAD)...).

L'invention concerne également une prothèse dentaire comprenant une source d'alimentation électrique couplée à un émetteur de signaux de radiocommunications apte à prendre un état faiblement actif, dans lequel il émet périodiquement des signaux à une première fréquence d'émission, et un état fortement actif, dans lequel il émet périodiquement des signaux à une deuxième fréquence d'émission supérieure à la première. Une telle prothèse comprend au moins un capteur de présence dans une bouche d'un utilisateur, apte à délivrer une information de présence ou d'absence de la prothèse dans la bouche ; l'émetteur est configuré pour passer de l'état faiblement actif à l'état fortement actif lorsque le capteur délivre une information d'absence de la prothèse dans la bouche, et pour prendre l'état faiblement actif sur détection d'une présence de la prothèse dentaire à proximité d'une base formant support de la prothèse dentaire lorsqu'elle n'est pas portée par un utilisateur.

L'invention concerne encore un procédé de traçabilité d'une prothèse dentaire, mis en œuvre dans le système de traçabilité décrit ci-avant.

### 4. Liste des figures

D'autres buts, caractéristiques et avantages de l'invention apparaîtront plus clairement à la lecture de la description suivante, donnée à titre de simple exemple illustratif, et non limitatif, en relation avec les figures, parmi lesquelles :
- la **figure 1** présente un synoptique général du système de traçabilité de prothèse dentaire selon un mode de réalisation de l'invention;
- la **figure 2** illustre un mode de fonctionnement du système de la figure 1 lorsqu'une prothèse dentaire est égarée;
- les **figures 3A et 3B** présentent respectivement des schémas synoptiques fonctionnels des modules électroniques embarqués dans la prothèse dentaire **(****figure 3B****)** et dans la base formant support de la prothèse **(****figure 3A****)** ;
- la **figure 4** présente sous forme de graphe d'états les différents états et modes de fonctionnement du système de la **figure 1** ;
- la **figure 5** illustre un exemple de forme d'un circuit imprimé à intégrer dans la prothèse dentaire selon un mode de réalisation de l'invention.

### 5. Description détaillée de modes de réalisation de l'invention

Le principe général de l'invention repose sur le couplage, dans une prothèse dentaire amovible, d'un émetteur de signaux de radiocommunication actif (i.e. alimenté par une source d'alimentation électrique, par opposition à un émetteur passif de type RFID), et d'un capteur de présence de la prothèse dentaire dans la bouche d'un utilisateur, qui permet de ne faire fonctionner l'émetteur de signaux de radiocommunication dans un mode consommateur en énergie, que lorsqu'on détecte que la prothèse dentaire n'est plus dans la bouche de son utilisateur, et est donc potentiellement égarée.

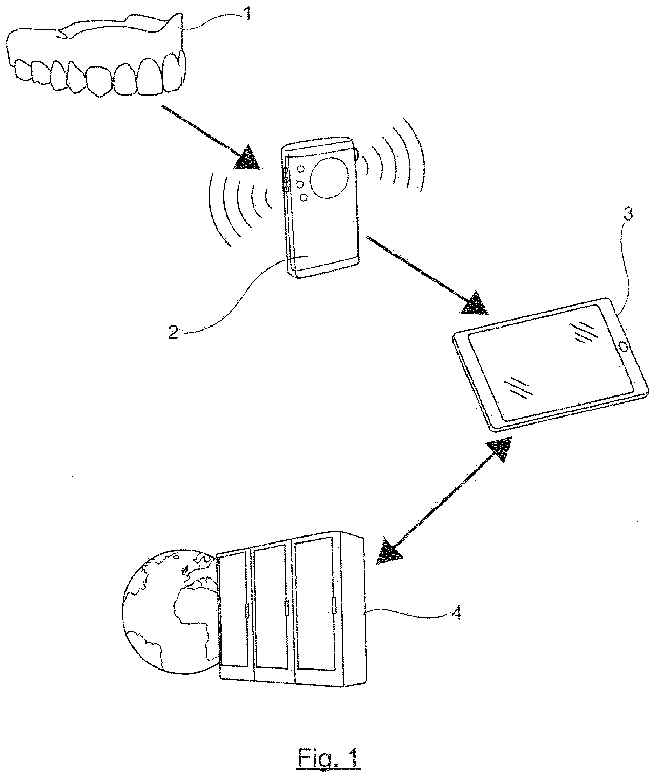

On présente désormais, en relation avec les **figures 1 à 4****,** le système et le procédé de traçabilité de prothèse dentaire selon différents modes de réalisation de l'invention.

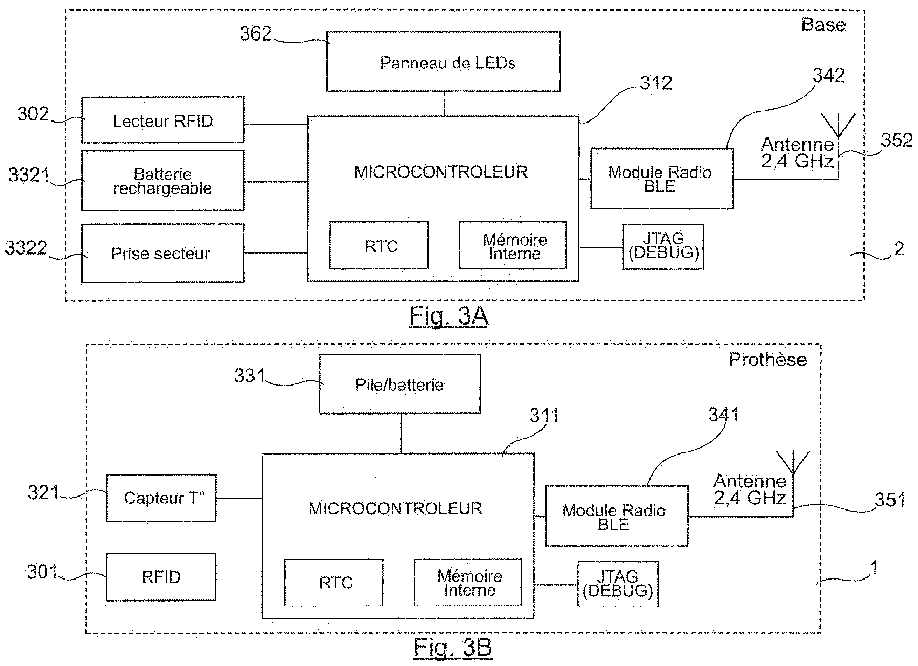

Comme illustré sur la **figure 1****,** un tel système de traçabilité comprend une prothèse dentaire amovible 1, qui peut être une prothèse totale ou partielle (dentier à crochet). Comme on le décrira plus en détail par la suite en relation avec la **figure 3B****,** une telle prothèse dentaire 1 embarque des composants électroniques, et notamment une puce active de type microcontrôleur, ainsi qu'une source d'alimentation électrique associée. Dans un mode de réalisation, elle intègre également une étiquette radiofréquence passive de type RFID. Ces différents composants permettent à la fois l'identification et la localisation de la prothèse dentaire 1, ainsi que la traçabilité des interventions et de l'entretien effectués sur la prothèse 1, mais aussi la communication de la prothèse 1 avec une interface de type téléphone intelligent ou tablette 3.

Une telle communication avec un téléphone intelligent ou une tablette 3 s'opère par l'intermédiaire d'un lecteur 2, comprenant un récepteur apte à recevoir les signaux de radiocommunication émis grâce à la puce active de la prothèse dentaire 1. Dans un mode de réalisation optionnel, le lecteur 2 comprend également un lecteur d'étiquette radiofréquence de type RFID, apte à lire les informations contenues dans l'étiquette RFID de la prothèse 1 lorsqu'il se situe à proximité de celle-ci.

Dans un mode de réalisation de l'invention, le lecteur 2 est constitué par une base formant support de la prothèse dentaire 1 lorsqu'elle n'est pas portée par son utilisateur. En variante, le lecteur 2 peut être un lecteur portatif, distinct de la base, mais apte à communiquer avec celle-ci. Le lecteur 2 peut également être intégré dans un dispositif de radiocommunication mobile tel qu'un téléphone intelligent ou une tablette.

Le lecteur 2 peut être en communication, via des signaux de type Bluetooth® par exemple, avec le support de communication 3 de type téléphone intelligent, tablette, voire-même robot-compagnon, dans le cas de personnes dépendantes, ou à capacités cognitives réduites. Une application logicielle peut être développée et exécutée sur le support de communication 3, pour former une articulation interactive avec le porteur de la prothèse dentaire 1 et/ou son entourage. Une telle communication peut être envisagée sur plusieurs plans :
- le support de communication 3, ou le lecteur 2 lui-même (bien que non illustré sur la **figure 1****),** peuvent faire remonter des informations vers un serveur 4. La gestion des données concernant la santé des personnes étant soumise à une réglementation liée à la protection des données, un tel serveur 4 est un serveur agréé permettant le stockage des données en provenance du support de communication 3 (comme celles du dossier médical patient), leur accès, leur communication, leur échange, conformément à la réglementation de la CNIL (Commission Nationale Informatique et Liberté - Loi française du 4 mars 2002). Un tel transfert d'informations vers le serveur 4 peut être effectué avec une certaine périodicité, par exemple à chaque fois que la prothèse dentaire 1 est reposée sur le lecteur 2 formant support. Notamment, le serveur 4 peut ainsi déterminer la durée qui s'écoule entre deux positionnements successifs de la prothèse dentaire 1 sur sa base 2. Si cette durée est trop importante, cela peut signifier que le patient n'entretient pas assez sa prothèse, ce qui peut être néfaste en termes d'hygiène, et entraîner l'apparition de lésions bucco-dentaires. Le serveur 4 peut également déterminer la durée de positionnement de la prothèse dentaire 1 sur sa base 2 : si cette durée est trop importante, cela peut signifier que la prothèse 1 n'est pas assez portée par son utilisateur, ce qui peut être en lien avec un inconfort, voire une zone de blessure des muqueuses buccales, provoquée par la prothèse dentaire 1. Le serveur 4 peut alors transmettre au téléphone intelligent ou à la tablette 3, pour affichage sur son écran, et consultation par l'utilisateur de la prothèse ou son entourage, des conseils sur les réglages de la prothèse dentaire 1 à effectuer en cabinet dentaire (réglage de l'articulé dentaire, rebasage/réajustage de la résine au contact des tissus...). Le serveur 4 peut également envoyer une alerte vers la base 2, qui peut clignoter, ou émettre un signal sonore pour signaler ce dysfonctionnement ou cette mauvaise utilisation de la prothèse ;
- le support de communication 3 peut permettre l'accès, à l'utilisateur de la prothèse 1 ou à son entourage, à une base de données informatives stockée sur le serveur 4. De telles données informatives peuvent être relatives à l'entretien de la prothèse (gestes d'hygiène, types de produits, méthodes de brossage...), aux visites de contrôle/réglage, au type d'alimentation à adapter en fonction des natures d'appareils, aux moyens de stabilisation (adhésif, attachements...), aux moyens de rendre la prothèse dentaire 1 plus confortable, aux méthodes d'adaptation à une nouvelle prothèse dentaire 1 dans les premiers jours de son utilisation...
- le support de communication 3 peut également permettre l'accès à des informations stockées sur le serveur 4, relatives à la fabrication de la prothèse dentaire 1 (normes ISO (« International Standardization Organisation » pour « Organisation Internationale de Normalisation »), types de matériaux, laboratoire de prothèse...)

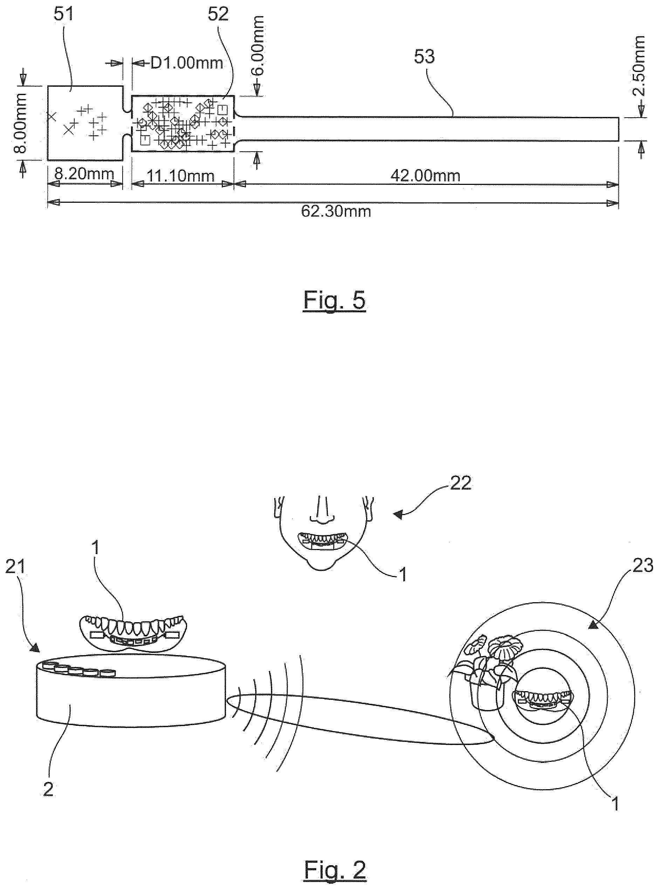

On présente désormais, en relation avec les **figures 2** **et** **3A****-3B,** le principe de fonctionnement du système de traçabilité de la **figure 1****,** lorsqu'une prothèse dentaire amovible 1 est égarée.

La **figure 2** illustre les trois positions principales de la prothèse dentaire amovible 1 :
- position 21 : positionnée sur sa base 2, formant support de la prothèse 1 lorsqu'elle n'est pas portée par son utilisateur, par exemple la nuit ;
- position 22 : dans la bouche de son utilisateur ;
- position 23 : égarée par son utilisateur.

En position référencée 21, le module radio BLE (Bluetooth Low Energy®) 342 (voir **figure 3A****)** intégré dans la base 2 peut adresser une requête d'obtention de données d'identification de la prothèse 1 et/ou du patient au microcontrôleur 311 de la prothèse 1 (voir **figure 3B**) : ces données sont stockées directement dans la mémoire interne du microcontrôleur 311, et peuvent être transmises à la base 2, par le module radio BLE 341 de la prothèse 1. Ces données permettent de relever une identification du propriétaire de la prothèse (numéro de matricule, tel que le numéro de sécurité sociale par exemple ou nom/prénom).

Dans une variante mettant en œuvre une étiquette radiofréquence passive RFID 301, en position référencée 21, le lecteur d'étiquettes radiofréquence 302 (voir **figure 3A**) intégré dans la base 2 permet une lecture à distance réduite de l'étiquette RFID 301 (voir **figure 3B**) intégrée dans la prothèse dentaire 1 : cette lecture permet d'accéder à ces mêmes données d'identification.

Ainsi, quand une prothèse dentaire 1 égarée est retrouvée, on peut, en la positionnant sur une base 2, vérifier l'identité de son propriétaire, ce qui est particulièrement utile en cas de perte dans une structure collective (milieu institutionnel, EHPAD, centres hospitaliers par exemple).

En outre, un tel échange de trames de données (requête et réponse) entre la base 2 et la prothèse 1 permet de renseigner la base 2 sur la présence à proximité de la prothèse dentaire 1. La base 2 détecte alors que la prothèse dentaire 1 doit fonctionner en mode « base », dans lequel le capteur de température 321 doit être dans un état inactif, afin de réduire la consommation d'énergie de la prothèse dentaire 1. Dans ce fonctionnement en mode « base », l'émetteur 341 de la prothèse 1 est également très peu actif, et n'émet par exemple qu'une trame BLE toutes les quinze minutes environ, afin de confirmer sa présence à proximité de la base 2. Sa consommation d'énergie est donc également très faible, de façon à préserver l'autonomie énergétique de la prothèse 1.

En effet, une problématique importante de ce type de système de traçabilité de prothèse dentaire consiste à assurer une autonomie énergétique suffisante de la prothèse, par exemple entre deux visites d'entretien successives chez le prothésiste dentaire, le plus souvent espacées de six mois. Il est donc particulièrement important, dans un mode de réalisation où la source d'alimentation électrique 331 (pile ou batterie) n'est pas rechargeable, de contrôler finement les consommations électriques des composants illustrés sur la **figure 3B****.** A cette fin, la puissance d'émission du module radio BLE 341 sera par exemple limitée à une puissance de 0 dBm.

Dans ce mode « base », lorsque la prothèse dentaire 1 se trouve en position référencée 21 sur la **figure 2****,** la consommation énergétique de la prothèse 1 est donc réduite au minimum : la transmission de signaux radiofréquence est très peu fréquente, aucune mesure de température n'est effectuée. En variante, l'émetteur 341 de la prothèse peut même être totalement désactivé, et le microcontrôleur 311 être en mode veille.

Lorsque le module radio BLE 341 ne reçoit plus de réponse de la base 2 aux trames émises, le microcontrôleur 311 détecte alors que la prothèse dentaire n'est plus positionnée 21 sur son support. L'hypothèse la plus probable est alors que la prothèse dentaire 1 est passée en position 22, dans la bouche de son utilisateur.

Le microcontrôleur 311 pilote alors le changement de fonctionnement de la prothèse dentaire 1 en mode « reconnaissance ». En variante, c'est le microcontrôleur 312 qui pilote l'émission, par le module radio BLE (Bluetooth Low Energy®) 342, d'un signal de commande destiné à faire passer la prothèse dentaire 1 en mode « reconnaissance ». Ce signal de contrôle est émis par l'antenne directive 352 de la base 2, et reçu par l'antenne 351 de la prothèse 1, puis décodé par le module radio BLE 341 de cette dernière.

Dans ce mode « reconnaissance », le microcontrôleur 311 de la prothèse dentaire 1 pilote alors l'activation du capteur de température 321 de la prothèse dentaire 1, et une mesure de température est alors opérée à intervalles de temps réguliers (par exemple toutes les cinq ou dix minutes) par le capteur de température 321. Le module radio BLE 341 de la prothèse 1 continue à émettre des trames BLE à intervalles de temps réguliers, mais très espacés, par exemple toutes les quinze minutes.

On notera qu'en variante, le capteur de température 321 peut être remplacé par exemple par un capteur d'humidité. De même, en variante, les modules radio 341 et 342 de la base 2 et de la prothèse 1 peuvent également utiliser d'autres protocoles de communication que le BLE, qui présente cependant l'avantage d'une portée satisfaisante de 20 à 30 mètres, d'une puissance d'émission relativement peu élevée, et d'une consommation énergétique satisfaisante. On pourrait cependant également envisager d'utiliser des protocoles de communication radio de type WiFi® (bien que présentant une consommation énergétique plus élevée que le BLE), NFC (pour « Near Field Communication », « communication en champ proche ») ou RFID active (ces deux derniers protocoles présentant cependant pour inconvénient une portée trop réduite et/ou la nécessité d'émission de multiples fréquences).

Lorsque la température mesurée par le capteur 321 est supérieure à un seuil de température configurable (par exemple 35°C), on considère que la prothèse 1 est portée par son propriétaire (position 22), et le mode « reconnaissance » reste enclenché. Le module radio BLE 341 fonctionne alors dans un mode peu consommateur en énergie, dans laquelle la fréquence d'émission des trames de données à destination de la base 2 est faible. En variante, dans ce mode « reconnaissance », la fonction radio du module radio BLE 341 est désactivée.

Lorsque la température mesurée par le capteur 321 descend au-dessous d'un certain seuil (par exemple 35°C), on considère que la prothèse 1 peut être égarée (position 23). Le microcontrôleur 311 de la prothèse dentaire 1 pilote alors le passage de la prothèse 1 dans un mode « écoute », dans lequel la fonction radio du module radio BLE 341 est activée mais maintenue à une consommation énergétique réduite (par exemple, émission d'une trame BLE toutes les deux minutes, au lieu de quinze en mode « reconnaissance »), en attente d'une réception d'informations de la base 2.

Lorsque le propriétaire de la prothèse 1, ou une personne de son entourage, détecte que la prothèse 1 a été égarée (position 23), il actionne un bouton ou un interrupteur prévu à cet effet sur la base 2. Dans une variante, cet actionnement ne se fait pas directement sur la base 2, mais par l'intermédiaire d'une application exécutée sur un téléphone mobile de type téléphone intelligent ou une tablette. Le microcontrôleur 312 de la base 2 pilote alors l'émission par le module radio BLE 342, et via l'antenne directive 352, de signaux de commande destinés à la prothèse 1, pour la faire passer en mode « localisation ». Dans ce mode de fonctionnement, le module radio BLE 341 de la prothèse 1 est actif et envoie périodiquement des signaux de radiocommunication destinés à la base 2 qui recherche la prothèse 1, et qui peuvent être captés par l'antenne directive 352 de la base 2 (par exemple, une trame BLE toutes les 30 secondes). La force du signal reçu par la base 2 est mesurée et corrélée à une distance et une direction pour permettre la géolocalisation de la prothèse 1 égarée.

L'antenne directive 352 de la base est par exemple une antenne type patch céramique, ou plusieurs (2 ou 4) antennes mises en réseau. En variante, l'antenne directive 352 peut consister en un réseau d'antennes de type patch sur PCB (pour « Printed Circuit Board », en français circuit imprimé).

Comme illustré sur la **figure 3A****,** la base 2 fonctionne sur alimentation secteur 3322 et intègre une batterie rechargeable 3321 permettant d'alimenter la base 2 lorsqu'elle est en itinérance, lorsque l'utilisateur est en recherche de la prothèse égarée.

Lorsque le signal émis par le module radio BLE 341 de la prothèse 1 est détecté par l'antenne directive 352 de la base 2, sans déplacement de l'utilisateur, un mouvement de rotation opéré par ce dernier permet d'optimiser la force du signal reçu, et donc de donner l'indication de sa direction de réception. Le déplacement de l'utilisateur permet de lui indiquer s'il se rapproche ou s'éloigne de la cible formée par la prothèse égarée. Un panneau d'indicateurs lumineux 362 facilite la recherche : par exemple, le nombre d'indicateurs lumineux éclairés augmente lorsque l'utilisateur pointe la base ou le lecteur portatif 2 dans la direction de réception du signal en provenance de la prothèse 1. En variante, le panneau d'indicateurs lumineux 362 peut être remplacé par un haut-parleur émettant des bips sonores de plus en plus rapprochés temporellement à mesure que la base 2 pointe dans la direction de la prothèse égarée 1. Cette variante est particulièrement avantageuse pour la recherche de la prothèse par un malvoyant, dont les investigations peuvent être guidées efficacement par cet accompagnement sonore.

Lorsque l'utilisateur, en suivant la direction indiquée par la base 2, retrouve la prothèse dentaire 1 égarée, il la positionne sur sa base 2, afin de lire les données d'identification qu'elle contient, et vérifier l'identité du propriétaire de la prothèse retrouvée. Si la prothèse 1 retrouvée correspond bien à la prothèse 1 recherchée, un échange de messages réussi entre le microcontrôleur 312 de la base 2 et le microcontrôleur 311 de la prothèse 1, par l'intermédiaire des modules radio BLE 341 et 342, permet de refaire passer la prothèse 1 en mode « base », dans lequel le capteur de présence 321 est dans un état inactif, et le microcontrôleur 311 est dans un état de faible consommation énergétique, pilotant l'émission à très faible fréquence de trames de données par le module radio BLE 341.

Le passage en mode « base» peut se faire à l'initiative du microcontrôleur 311 de la prothèse dentaire 1, sur détection d'une réponse du module radio BLE 342 de la base 2 à une requête émise par le module radio BLE 341 de la prothèse dentaire 1. Il peut également être piloté par le module radio BLE 342 de la base 2, qui émet un signal de commande de fonctionnement de la prothèse dentaire 1 en mode « base » : à réception de ce signal de commande, le microcontrôleur 311 de la prothèse pilote la désactivation du capteur de présence 321, et réduit la fréquence d'émission de trames de données par le module radio BLE 341 à une trame toutes les quinze minutes environ.

Si la prothèse 1 est trop éloignée de la base (typiquement au-delà de 25 mètres), aucun signal n'est détecté par la base 2. Un message d'alerte (voyant lumineux, clignotant, alarme sonore) est diffusé sur la base 2 lorsque l'utilisateur a initié le passage en mode « localisation », pour lui signaler cette situation. L'utilisateur doit alors déplacer la base 2 jusqu'à ce qu'elle se trouve à portée radio de la prothèse égarée.

Les différents composants électroniques de la prothèse 1 illustrés en **figure 3B** peuvent être disposés en plusieurs emplacements (trois par exemple) de la prothèse dentaire 1, ainsi qu'illustré en **figure 5****.** Le volume disponible de chaque logement peut être de l'ordre de 1cm x 0,5cm x 3mm, et ces différentes zones ou logements peuvent être reliées entre elles par connexion filaire. Ces logements sont réalisés au laboratoire de prothèse dentaire : le prothésiste réalise un fraisage dans la masse de résine de la prothèse, met en place les matériels électroniques, et les scelle dans leur cavité respective par l'adjonction d'une résine fluide auto-polymérisable ou photo-polymérisable, de façon à isoler les composants électroniques du milieu buccal, humide et bactérien. La pile 331 peut consister par exemple en deux piles PR63 de 5,8 mm de diamètre et 2,1 mm d'épaisseur.

Le schéma de la **figure 5** illustre un exemple de forme du circuit imprimé à intégrer dans la prothèse dentaire. Un tel circuit imprimé comprend trois zones référencées 51 à 53. Les zones référencées 51 et 53 sont de préférence flexibles, tout particulièrement la zone longue référencée 53, qui est particulièrement souple. Les symboles en croix et en carrés illustrent l'implantation des composants électroniques, qui sont présents sur les zones référencées 51, et plus particulièrement 52. Ainsi, la présence en nombre de composants dans la zone centrale 52 permet de rigidifier cette partie du circuit imprimé. Les dimensions en millimètres mentionnées sur le schéma de la **figure 5** donnent une indication de la taille réelle d'un tel circuit imprimé.

Dans une variante de réalisation, la source d'alimentation électrique 331 de la prothèse dentaire est rechargeable. Par exemple, son rechargement s'opère par inductance, lorsque la prothèse 1 est positionnée sur la base 2, par exemple pendant la nuit après nettoyage de l'appareil.

En outre, la base 2 peut présenter un témoin d'usure de la pile ou batterie 331, afin d'alerter l'utilisateur de la prothèse sur la nécessité de recharger ou de changer la pile ou la batterie 331. Par exemple, un tel témoin est un voyant lumineux, qui s'éclaire en vert lorsque le niveau de charge de la pile ou batterie 331 est supérieur ou égale à 30%, en orange lorsqu'il est compris entre 15% et 30%, et en rouge lorsqu'il devient inférieur à 15%.

En variante, le microcontrôleur 311 de la prothèse peut piloter l'émission d'un signal d'indication d'usure de la pile ou batterie 331 vers le téléphone intelligent ou la tablette 3, pour prévenir l'utilisateur ou son entourage que le niveau de charge de la pile ou batterie 331 est faible.

La base 2 peut également être équipée d'un compteur ou d'une horloge, permettant d'évaluer le temps restant avant la prochaine visite de contrôle et d'entretien chez le prothésiste dentaire ou le chirurgien-dentiste. De telles visites doivent généralement avoir lieu tous les six mois environ. Dans un mode de réalisation, lorsque le compteur de la base 2 indique qu'il ne reste plus qu'un mois avant la prochaine visite de contrôle et d'entretien, le microcontrôleur 312 de la base 2 pilote l'émission d'un signal d'alerte vers le téléphone intelligent ou la tablette 3 pour prévenir l'utilisateur ou son entourage qu'il est temps de prendre rendez-vous chez le prothésiste dentaire ou le chirurgien-dentiste. Une telle visite chez le prothésiste dentaire peut être l'occasion de remplacer la pile 331 de la prothèse 1, et de réinitialiser le compteur/horloge de la base 2. En variante, l'alerte est directement émise par la base 2, sur un écran dédié, ou par allumage d'un indicateur lumineux, ou encore par émission d'un message sonore.

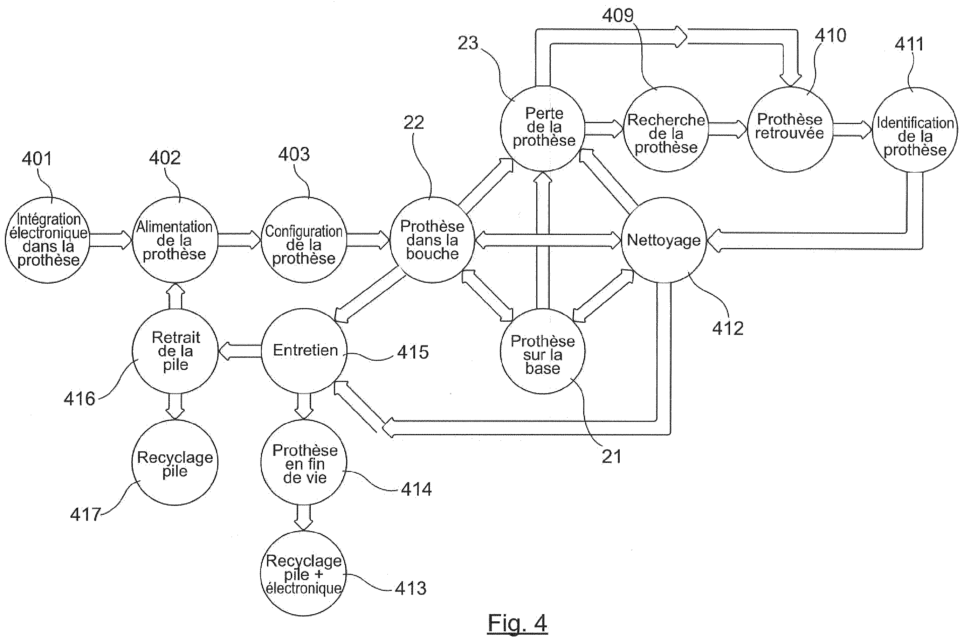

La **figure 4** présente sous forme de diagramme d'états les différents états possibles du système de traçabilité selon un mode de réalisation de l'invention. Sur cette figure, les différents cercles indiquent les états possibles du système de traçabilité selon l'invention, tandis que les flèches représentent les passages possibles d'un état à un autre. Plus particulièrement :
- l'état référencé 401 correspond à l'intégration de l'ensemble des composants électroniques de la **figure 3B** dans la prothèse dentaire 1 ;
- à l'issue de l'état référencé 401, on passe dans l'état référencé 402, dans lequel la prothèse dentaire est alimentée électriquement, par exemple par insertion de la pile 331 ;
- à partir de là, on peut procéder à la configuration 403 de la prothèse, par exemple en mémorisant dans l'étiquette RFID 301, ou dans la mémoire interne du microcontrôleur 311, les données d'identification du propriétaire de la prothèse (n° de sécurité sociale, nom/prénom...) et en mémorisant dans la mémoire interne du microcontrôleur 311 les instructions de code de programme nécessaires au fonctionnement du système de traçabilité de l'invention ;

- après configuration 403 de la prothèse, la prothèse peut passer dans l'état référencé 22, où elle se trouve dans la bouche de son propriétaire ;
- de cet état 22 de prothèse en bouche, différents scenarii peuvent être envisagés :
   ∘ la prothèse 1 peut passer dans l'état 21 où la prothèse est positionnée sur sa base 2 ;
   ∘ la prothèse 1 peut être égarée (état référencé 23) ;
   ∘ la prothèse 1 peut passer dans un état 412 de nettoyage (brossage, positionnement dans un verre d'eau, avec adjonction éventuelle de produits d'entretien...) ;
   ∘ enfin, la prothèse 1 peut repasser dans un état 415 d'entretien, chez le prothésiste dentaire, par exemple pour un réglage ou un ajustement de la prothèse 1 à la morphologie de son utilisateur.

De l'état 21 où la prothèse 1 est sur sa base 2, il est également possible de passer dans l'état 22 où la prothèse est en bouche, ou dans l'état 412 de nettoyage de la prothèse, ou encore dans l'état 23 où la prothèse est perdue. Quand la prothèse 1 quitte l'état 21, le capteur de présence passe dans l'état actif.

Il est également possible de passer directement d'un état 412 de nettoyage de la prothèse 1 à un état 415 d'entretien chez le prothésiste dentaire. A l'issue du nettoyage 412, il est aussi possible de perdre la prothèse (état 23).

Lorsque la prothèse est perdue (état 23), elle peut être directement retrouvée par son utilisateur (état 410), par exemple parce qu'elle est visuellement accessible à l'utilisateur. Si l'utilisateur ne la localise pas, il peut utiliser la base 2 pour lancer une recherche active 409 de la prothèse dentaire.

Dans tous les cas, lorsque la prothèse dentaire est retrouvée (état 410), il est souhaitable de procéder à l'identification de son propriétaire (état 411), par lecture des données contenues dans l'étiquette RFID 301 de la prothèse, ou obtention de ces données mémorisées dans le microcontrôleur 311 en réponse à une requête émise par le module radio BLE 342 de la base 2. Cet accès aux données d'identification de la prothèse et/ou du patient peut se faire au moyen de la base 2, ou d'un lecteur portatif dédié, ou encore au moyen d'un téléphone intelligent ou d'une tablette équipés d'un lecteur RFID. On peut aussi envisager que l'étiquette RFID ou la mémoire interne du microcontrôleur 311 ne contienne qu'un numéro d'identification, et que le lecteur (base 2, téléphone intelligent, ou tout autre équipement de lecture approprié) se connecte au serveur de données 4 pour accéder à une base de données mémorisant en association l'ensemble des numéros d'identification, et les identités de leurs propriétaires (par exemple nom/prénom, et dans le cas d'une vie en structure collective, numéro de chambre et étage).

Après identification 411 du propriétaire de la prothèse dentaire 1 retrouvée, il est impératif, pour des questions d'hygiène, de repasser dans l'état 412 de nettoyage de la prothèse dentaire 1.

Lors des visites semestrielles chez le prothésiste dentaire (état 415), ce dernier retire la pile 331 de la prothèse dentaire 1 pour la changer. La pile usagée est recyclée (état 417) et une nouvelle pile est insérée dans la prothèse dentaire (état 402), en remplacement de la pile usagée.

Lors de ces visites, il est aussi possible que le prothésiste détecte qu'il est temps de changer la prothèse dentaire : la prothèse passe alors dans un état de fin de vie 414. La pile 331 et l'électronique embarquée dans la prothèse 1 peuvent alors être recyclées (état 413).

## Revendications

1. Système de traçabilité d'une prothèse dentaire (1) comprenant la prothèse dentaire (1), ladite prothèse dentaire comprenant une source d'alimentation électrique (331) couplée à un émetteur (341) de signaux de radiocommunications apte à prendre un état faiblement actif, dans lequel il émet périodiquement des signaux à une première fréquence d'émission, et un état fortement actif, dans lequel il émet périodiquement des signaux à une deuxième fréquence d'émission supérieure à la première, et au moins un capteur (321) de présence de ladite prothèse dans une bouche d'un utilisateur (22), apte à délivrer une information de présence ou d'absence de ladite prothèse dans ladite bouche,
**caractérisé en ce que** ledit émetteur (341) de signaux de radiocommunication est configuré pour passer dudit état faiblement actif audit état fortement actif lorsque ledit capteur délivre une information d'absence de ladite prothèse dans ladite bouche,
**et en ce qu'**il comprend une base (2) formant support de ladite prothèse dentaire lorsqu'elle n'est pas portée par un utilisateur, **et en ce que** ledit émetteur est configuré pour prendre ledit état faiblement actif sur détection d'une présence de ladite prothèse dentaire à proximité de ladite base.

2. Système selon la revendication 1, **caractérisé en ce que** sur détection d'une présence de ladite prothèse dentaire (1) à proximité de ladite base (2), ladite prothèse dentaire est configurée pour fonctionner en mode « base », dans lequel ledit capteur de présence est dans un état inactif et dans lequel ledit émetteur de signaux de radiocommunications est dans ledit état faiblement actif.

3. Système selon l'une quelconque des revendications 1 et 2, **caractérisé en ce que** sur détection d'une absence de ladite prothèse dentaire (1) à proximité de ladite base (2), ladite prothèse dentaire est configurée pour fonctionner en mode « reconnaissance », dans lequel ledit capteur de présence opère une mesure de présence à une fréquence déterminée, et dans lequel ledit émetteur de signaux de radiocommunications est dans ledit état faiblement actif.

4. Système selon l'une quelconque des revendications 1 à 3, **caractérisé en ce que** ledit émetteur est configuré pour passer, lorsque ledit capteur délivre une information d'absence de ladite prothèse dans ladite bouche, dudit état faiblement actif audit état fortement actif dans lequel il est configuré pour émettre périodiquement des signaux à une deuxième fréquence d'émission,
**et en ce que** ladite base comprend un module d'émission d'une commande d'un mode de fonctionnement de ladite prothèse dentaire, configuré pour envoyer à ladite prothèse dentaire, sur action d'un utilisateur, une commande de fonctionnement en mode « localisation», dans lequel ledit émetteur de signaux de radiocommunication est configuré pour émettre périodiquement des signaux à une troisième fréquence d'émission supérieure à la deuxième.

5. Système selon l'une quelconque des revendications 1 à 4, **caractérisé en ce que** ladite base comprend un module d'alerte configuré pour émettre une alerte :
- lorsqu'une durée de présence de ladite prothèse dentaire à proximité de ladite base est supérieure à un seuil de présence déterminé, ou
- lorsqu'une durée d'absence de ladite prothèse dentaire à proximité de ladite base est supérieure à un seuil d'absence déterminé.

6. Système selon l'une quelconque des revendications 1 à 5, **caractérisé en ce que** ladite base (2) comprend une unité de localisation de ladite prothèse dentaire, comprenant au moins une antenne directive (352) connectée à un module radio (342) aptes à recevoir les signaux de radiocommunication émis par l'émetteur de ladite prothèse dentaire, et un ensemble (362) d'indicateurs lumineux de contrôle d'une direction de réception desdits signaux.

7. Système selon l'une quelconque des revendications 1 à 6, **caractérisé en ce que** ladite base comprend un module de chargement par induction de ladite source d'alimentation électrique.

8. Système selon l'une quelconque des revendications 1 à 7, **caractérisé en ce en ce que** ladite prothèse dentaire comprend une étiquette d'identification radiofréquence passive (301), **et en ce que** ladite base comprend un lecteur (302) d'étiquette d'identification radiofréquence.

9. Prothèse dentaire (1) comprenant une source d'alimentation électrique (331) couplée à un émetteur (341) de signaux de radiocommunications apte à prendre un état faiblement actif, dans lequel il émet périodiquement des signaux à une première fréquence d'émission, et un état fortement actif, dans lequel il émet périodiquement des signaux à une deuxième fréquence d'émission supérieure à la première, et au moins un capteur (321) de présence dans une bouche d'un utilisateur, apte à délivrer une information de présence ou d'absence de ladite prothèse dans ladite bouche,
**caractérisée en ce que** ledit émetteur est configuré pour passer dudit état faiblement actif audit état fortement actif lorsque ledit capteur délivre une information d'absence de ladite prothèse dans ladite bouche,
**et en ce qu'**il est configuré pour prendre ledit état faiblement actif sur détection d'une présence de ladite prothèse dentaire à proximité d'une base (2) formant support de ladite prothèse dentaire lorsqu'elle n'est pas portée par un utilisateur.

10. Procédé de traçabilité d'une prothèse dentaire, comprenant une source d'alimentation électrique (331) couplée à un émetteur (341) de signaux de radiocommunications apte à prendre un état faiblement actif, dans lequel il émet périodiquement des signaux à une première fréquence d'émission, et un état fortement actif, dans lequel il émet périodiquement des signaux à une deuxième fréquence d'émission supérieure à la première,
le procédé comprenant:
- une étape de fourniture, par au moins un capteur (321) de présence de ladite prothèse dans une bouche d'un utilisateur, d'une information d'absence de ladite prothèse dans ladite bouche,
- sur réception de ladite information d'absence, une étape de passage dudit émetteur dudit état faiblement actif audit état fortement actif,
- une étape de détection d'une présence de ladite prothèse dentaire à proximité d'une base (2) formant support de ladite prothèse dentaire lorsqu'elle n'est pas portée par un utilisateur,
- sur détection de présence de ladite prothèse dentaire à proximité de ladite base, une étape de configuration dudit émetteur dans ledit état faiblement actif.

## Patentansprüche

1. System zur Rückverfolgung einer Zahnprothese (1), umfassend die Zahnprothese (1), wobei der Zahnprothese eine elektrische Versorgungsquelle (331) umfasst, die an einen Sender (341) von Funksignalen gekoppelt ist, der geeignet ist, einen schwach aktiven Zustand, in dem er regelmäßig Signale auf einer ersten Sendefrequenz sendet, und einen stark aktiven Zustand einzunehmen, in dem er regelmäßig Signale auf einer zweiten Sendefrequenz sendet, die höher als die erste ist, und mindestens einen Anwesenheitssensor (321) der Zahnprothese in einem Mund eines Benutzers (22), der geeignet ist, eine Anwesenheits- oder Abwesenheitsinformation der Zahnprothese im Mund zu liefern,
**dadurch gekennzeichnet, dass** der Sender (341) von Funksignalen dazu eingerichtet ist, vom schwach aktiven Zustand in den stark aktiven Zustand überzugehen, wenn der Sensor eine Abwesenheitsinformation der Zahnprothese vom Mund liefert,
und dass es eine Basis (2) umfasst, die einen Träger der Zahnprothese bildet, wenn er von einem Benutzer nicht getragen wird, und dass der Sender eingerichtet ist, um den schwach aktiven Zustand einzunehmen, wenn die Anwesenheit der Zahnprothese in der Nähe der Basis erfasst wird.

2. System nach Anspruch 1, **dadurch gekennzeichnet, dass** die Zahnprothese dazu eingerichtet ist, bei Erfassung einer Anwesenheit des Zahnprothese (1) in der Nähe der Basis (2) im "Basismodus" zu funktionieren, in welchem der Anwesenheitssensor in einem inaktiven Zustand ist, und in dem der Sender von Funksignalen im schwach aktiven Zustand ist.

3. System nach einem der Ansprüche 1 und 2, **dadurch gekennzeichnet, dass** die Zahnprothese dazu eingerichtet ist, bei Erfassung eines Fehlens des Zahnprothese (1) in der Nähe der Basis (2) im "Erkennungsmodus" zu funktionieren, in dem der Anwesenheitssensor eine Anwesenheitsmessung auf einer bestimmten Frequenz durchführt, und in dem der Sender von Funksignalen im schwach aktiven Zustand ist.

4. System nach einem der Ansprüche 1 bis 3, **dadurch gekennzeichnet, dass** der Sender dazu eingerichtet ist, vom schwach aktiven Zustand in den stark aktiven Zustand überzugehen, wenn der Sensor eine Information über ein Fehlen der Zahnprothese im Mund liefert in welchem er dazu eingerichtet ist, regelmäßig Signale auf einer zweiten Sendefrequenz zu senden,
und dass die Basis ein Modul zum Senden einer Steuerung eines Funktionsmodus der Zahnprothese umfasst, das eingerichtet ist, an der Zahnprothese bei Betätigung durch einen Benutzer eine Steuerung einer Funktion im "Lokalisierungsmodus" zu senden, in welchem der Sender von Funksignalen dazu eingerichtet ist, regelmäßig Signale auf einer dritten Sendefrequenz zu senden, die höher als die zweite ist.

5. System nach einem der Ansprüche 1 bis 4, **dadurch gekennzeichnet, dass** die Basis ein Alarmmodul umfasst, das dazu eingerichtet ist, einen Alarm zu senden:
- wenn eine Dauer der Anwesenheit der Zahnprothese in der Nähe der Basis länger als eine vorbestimmte Anwesenheitsdauer ist, oder
- wenn eine Dauer des Fehlens der Zahnprothese in der Nähe der Basis länger als eine vorbestimmte Fehldauer ist.

6. System nach einem der Ansprüche 1 bis 5, **dadurch gekennzeichnet, dass** die Basis (2) eine Einheit zur Lokalisierung der Zahnprothese umfasst, umfassend mindestens eine Richtantenne (352), die an ein Funkmodul (342) angeschlossen ist, die dazu geeignet sind, die Funksignale zu empfangen, die vom Sender der Zahnprothese gesandt werden, und eine Gesamtheit (362) von Leuchtanzeigen zur Kontrolle einer Empfangsrichtung der Signale.

7. System nach einem der Ansprüche 1 bis 6, **dadurch gekennzeichnet, dass** die Basis ein Induktionslademodul der elektrischen Versorgungsquelle umfasst.

8. System nach einem der Ansprüche 1 bis 7, **dadurch gekennzeichnet, dass** die Zahnprothese ein passives Funkfrequenz-Identifikationsetikett (301) umfasst, und dass die Basis einen Leser (302) eines Funkfrequenz-Identifikationsetiketts umfasst.

9. Zahnprothese (1), umfassend eine elektrische Versorgungsquelle (331), die an einen Sender (341) von Funksignalen gekoppelt ist, der geeignet ist, einen schwach aktiven Zustand, in dem er regelmäßig Signale auf einer ersten Sendefrequenz sendet, und einen stark aktiven Zustand einzunehmen, in dem er regelmäßig Signale auf einer zweiten Sendefrequenz, die höher als die erste ist, sendet,
und mindestens einen Anwesenheitssensor (321) der Zahnprothese in einem Mund eines Benutzers, der dazu geeignet ist, eine Anwesenheits- oder Abwesenheitsinformation der Zahnprothese im Mund zu liefern,
**dadurch gekennzeichnet, dass** der Sender dazu eingerichtet ist, vom schwach aktiven Zustand in den stark aktiven Zustand überzugehen, wenn der Sensor eine Abwesenheitsinformation der Zahnprothese vom Mund liefert,
und dass er dazu eingerichtet ist, den schwach aktiven Zustand bei Erfassung einer Anwesenheit der Zahnprothese in der Nähe einer Basis (2), die einen Träger der Zahnprothese bildet, einzunehmen, wenn er nicht von einem Benutzer getragen wird.

10. Verfahren zur Rückverfolgung einer Zahnprothese, umfassend eine elektrische Versorgungsquelle (331), die an einen Sender (341) von Funksignalen gekoppelt ist, der geeignet ist, einen schwach aktiven Zustand, in dem er regelmäßig Signale auf einer ersten Sendefrequenz sendet, und einen stark aktiven Zustand einzunehmen, in dem er regelmäßig Signale auf einer zweiten Sendefrequenz, die höher als die erste ist, sendet, wobei das Verfahren umfasst:
- einen Schritt der Lieferung einer Abwesenheitsinformation der Zahnprothese vom Mund durch mindestens einen Sensor (321) zur Erfassung der Anwesenheit der Zahnprothese in einem Mund eines Benutzers,
- einen Schritt des Übergangs des Senders vom schwach aktiven Zustand in den stark aktiven Zustand, bei Empfang der Abwesenheitsinformation
- einen Schritt der Erfassung einer Anwesenheit der Zahnprothese in der Nähe einer Basis (2), die einen Träger für die Zahnprothese bildet, wenn er nicht vom Benutzer getragen wird,
- bei Erfassung einer Anwesenheit der Zahnprothese in der Nähe der Basis einen Schritt der Einrichtung des Senders im schwach aktiven Zustand.

## Claims

1. System of traceability of a dental prosthesis (1) comprising the dental prosthesis (1),
said dental prosthesis (1) comprising an electrical power source (331) coupled with a radio-communication signals transmitter (341) capable of assuming a weakly active state in which it periodically sends out signals at a first transmission frequency, and a highly active state in which it periodically sends out signals at a second transmission frequency higher than the first transmission frequency, and at least one presence sensor (321) for sensing the presence of the prosthesis in a mouth of a user (22), capable of delivering a piece of information on the presence or absence of said prosthesis in said mouth,
**characterized in that** said radio-communication signals transmitter (341) is configured to pass from said weakly active state to said highly active state when said sensor delivers a piece of information on absence of the prosthesis in said mouth,
**and in that** it comprises a base (2) forming a support of said dental prosthesis when it is not being worn by a user, **and in that** said transmitter is configured to assume said weakly active state on detecting a presence of said dental prosthesis in proximity to said base.

2. System according to claim 1 **characterized in that,** on detecting a presence of said dental prosthesis (1) in proximity to said base (2), said dental prosthesis is configured to operate in "base" mode in which said presence sensor is in an inactive state and in which said radio-communication signals transmitter is in a weakly active state.

3. System according to any one of the claims 1 and 2 **characterized in that,** on detecting an absence of said dental prosthesis (1) in proximity to said base (2), said dental prosthesis is configured to work in "recognition" mode in which said presence sensor carries out a measurement of presence at a determined frequency, and in which said radio-communication signals transmitter is in said weakly active state.

4. System according to any one of the claims 1 to 3, **characterized in that** said transmitter is configured so that, when said sensor delivers a piece of information on absence of said prosthesis in said mouth, it passes from said weakly active state to said highly active state in which it is configured to periodically send out signals at a second transmission frequency,
**and in that** said base comprises a module for sending a command for a mode of operation of said dental prosthesis that is configured to send, to said dental prosthesis, upon action by a user, a command for operation in "location" mode, in which said radio-communication signals transmitter is configured to periodically send out signals at a third transmission frequency higher than the second transmission frequency.

5. System according to any one of the claims 1 to 4, **characterized in that** said base comprises a warning module configured to send out an alert:
- when a duration of presence of said dental prosthesis in proximity to said base is greater than a determined threshold of presence, or
- when a duration of absence of said dental prosthesis in proximity to said base is greater than a determined threshold of absence.

6. System according to any one of the claims 1 to 5, **characterized in that** said base (2) comprises a unit for locating said dental prosthesis, comprising at least one directional antenna (352) connected to a radio module (342) capable of receiving radio-communication signals sent out by the transmitter of said dental prosthesis and a set (362) of light indicators for controlling a direction of reception of said signals.

7. System according to any one of the claims 1 to 6, **characterized in that** said base comprises a module for the charging of said electrical power source by induction.

8. System according to any one of the claims 1 to 7, **characterized in that** said dental prosthesis comprises a passive radiofrequency identification label (301) **and in that** said base comprises a radiofrequency identification label reader (302).

9. Dental prosthesis (1) comprising an electrical power supply source (331) coupled with a radio-communication signals transmitter (341) capable of assuming a weakly active state in which it periodically sends out signals at a first transmission frequency and a highly active state in which it periodically sends out signals at a second transmission frequency higher than the first frequency,
and at least one presence sensor (321) for sensing presence in a user's mouth, capable of delivering a piece of information on presence or absence of said prosthesis in said mouth,
**characterized in that** said transmitter is configured to pass from said weakly active state to said highly active state when said sensor delivers a piece of information on absence of said prosthesis in said mouth,
**and in that** it is configured to assume said weakly active state on detecting a presence of said dental prosthesis in proximity to a base (2) forming a support of said dental prosthesis when it is not being worn by a user.

10. Method of traceability of a dental prosthesis, comprising an electrical power source (331) coupled with a radio-communication signals transmitter (341) capable of assuming a weakly active state in which it periodically sends out signals at a first transmission frequency, and a highly active state in which it periodically sends out signals at a second transmission frequency higher than the first transmission frequency,
the method comprising :
- a step for supplying, by means of at least one presence sensor (321) for sensing the presence of said prosthesis in a mouth of a user, a piece of information on absence of said prosthesis in said mouth,
- upon receiving said piece of information on absence, a step for passing from said weakly active state to said highly active state,
- a step for detecting a presence of said dental prosthesis in proximity to a base (2) forming a support of said dental prosthesis when it is not being worn by a user,
- on detecting a presence of said dental prosthesis in proximity to said base, a step for configuring said transmitter in said weakly active state.
